(19) Europäisches Patentamt
European Patent Office
Office européen des brevets

(11) Veröffentlichungsnummer: **0 219 491 B1**

# (12) EUROPÄISCHE PATENTSCHRIFT

(45) Veröffentlichungstag der Patentschrift: **30.03.88**

(51) Int. Cl.⁴: **A 61 K 31/435**, C 07 D 471/20, C 07 D 491/22 // (C07D471/20, 221:00, 209:00, 209:00),(C07D491/22, 311:00, 221:00, 209:00, 209:00)

(21) Anmeldenummer: **84902690.1**

(22) Anmeldetag: **06.07.84**

(86) Internationale Anmeldenummer: **PCT/AT 84/00025**

(87) Internationale Veröffentlichungsnummer: **WO 86/00524 (30.01.86** Gazette **86/3)**

## (54) OXINDOLALKALOIDE MIT IMMUNSYSTEMSTIMULIERENDEN EIGENSCHAFTEN UND DIESE ENTHALTENDES PRÄPARAT.

(43) Veröffentlichungstag der Anmeldung: **29.04.87 Patentblatt 87/18**

(45) Bekanntmachung des Hinweises auf die Patenterteilung: **30.03.88 Patentblatt 88/13**

(84) Benannte Vertragsstaaten: **AT BE CH DE FR GB LI LU SE**

(56) Entgegenhaltungen:
**GB - A - 1 056 537**
**GB - A - 1 056 863**

**Chemical Abstracts, Band 92, 9. Juni 1980, Columbus, Ohio (US),Chang, Ting-Hsin et al.: "Hypotensive effect of rhynchopylla total alkaloids and rhynchophylline", siehe Seite 59, Zusammenfassung 191363c**
**Chemical Abstracts, Band 91, 13. August 1979, Columbus, Ohio (US),H. Masatoshi et al.: "Effects of indole alkaloids from Gardneria nutans Sieb. and Zucc. and Uncaria rhynchophylla Miq. on a guinea pig urinary bladder preparation in situ", siehe Seite 55, Zusammenfassung 49608x**
**Chemical Abstracts, Band 84, 1976, Columbus, Ohio (US),H. Masatoshi et al.: "Effect of indole alkaloids from Gardneria genus and Uncaria genus on neuromuscular transmission in the rat limb in situ" siehe Seite 50, Zusammenfassung 144859u**
**Chemical Abstracts, Band 91, 24. September 1979, Columbus, Ohio (US),Chang, Chuan-Chiung et al.: "Study on the antihypertensive action of uncarine A, an alkaloid of Uncaria formosana used in Chinese herb medicine", siehe Seite 53, Zusammenfassung 102109p**
**Chemical Abstracts, Band 90, 9. April 1979, Columbus, Ohio (US),C.C. Chang et al.: "Study on the mode of hypotensive action of Uncarine A.", siehe Seite 75, Zusammenfassung 115313e**

(73) Patentinhaber: **KEPLINGER, Klaus, Müllerstrasse 30, A-6020 Innsbruck (AT)**

(72) Erfinder: **WAGNER, Hildebert, Nelkenweg 9, D-8211 Breitbrunn am Chiemsee (DE)**
Erfinder: **KREUTZKAMP, Barbara, Conrad-Celtis-Strasse 20, D-8000 München 40 (DE)**

(74) Vertreter: **Torggler, Paul, Dr. et al, Patentanwälte Dr. Paul Torggler Dr. Engelbert Hofinger Wilhelm-Greil-Strasse 16, A-6020 Innsbruck (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft die Verwendung zumindest eines der pentacyclischen Oxindolalkaloide Isopteropodin, Pteropodin, Isomitraphyllin und Mitraphyllin zur Herstellung vom immunsystemstimulierenden Präparaten.

Des weiteren betrifft die Erfindung ein zumindest ein derartiges Oxindolalkaloid enthaltenes Präparat mit Ausnahme eines weitgehend gerbstoffreien Extraktes aus Wurzelteilen der Uncaria tomentosa (WILLD.) DC, deren frischer Bast gelbbraum oder dunkelrot gefärbt ist.

Trotz grosser Fortschritte in der Therapie von Infektionskrankheiten und Tumorerkrankungen durch Antibiotika und Cytostatika haben therapieresistente Infektionskrankheiten und opportunistische Infektionen zugenommen. Wir verfügen über wenig brauchbare Virustatika und die Therapie mit Cytostatika ist mit dem Handicap der hiermit zwangsläufig verbundenen Immunsuppression belastet. Diese Situation hat zu einer intensiven Suche nach alternativen bzw. adjuvatorischen Behandlungsmethoden geführt. Ein Behandlungskonzept, früher unter dem Begriff der Reit- und Umstimmungstherapie, heute als Immunstimulation bekannt, ist daher wieder aktuell geworden. Dabei interessiert vor allem die primär unspezifische Steigerung humoraler und zellulärer Abwehrmechanismen durch sogenannte Immunstimulantien, also durch Stoffe oder Stoffgemische, die spezifische und unspezifische körpereigene Abwehrmechanismen induzieren oder die Immunantwort auf ein gliechzeitig mitverabreichtes Antigen verstärken.

Als Prototyp mit erwiesener immunstimulierender Wirkung kann Aristolochiasäure (AS) gelten. Aristolochia clematitis (Aristolochiaceae) wurde schon von den alten Ägypten und Griechen bei allen Arten der Wundinfektion eingesetzt. Es wurden Versuchsmodelle entwickelt (J.R. Möse und G. Lukas, Arzneimittelforschung 11, 33, 1961), mit denen eine Stimulierung der körpereingenen Abwehrmöglichkeiten des Organismus nachweisbar ist. Als Testextrakt wurde ein nach den Vorschriften des Homöopathischen Arzneibuchs hergestellter Auszug aus Aristolochia clematitis gewählt. Bei der Untersuchung der Phagozytoseaktivität von Kaninchenleukozyten stellte sich eine Steigerung der Phagozytoseaktivität auf das Doppelte des Ausgangswertes ein, nachdem die Tiere über mehrere Tage verschiedene Verdünnungen des Aristolochiaauszuges i.v. injiziert bekommen hatten. Die Verdünnung D3 war dabei am wirksamsten. Ab der Verdünnung D7 war keine Wirkung mehr zu erkennen.

Aus der WO-A-82/1130 ist bekannt geworden, dass weitgehend gerbstoffreie Wurzelextrakte der Uncaria tomentosa (WILLD.) DC mit gelbbrauner oder dunkelroter Färbung des frischen Bastes als Antitumormittel, Kontrazeptivum oder entzündungshemmendes Mittel einsetzbar sind. Auf Grund der darin beschriebenen Herstellungsverfahren, die zum Teil Isolierverfahren von pflanzlichen Alkaloiden sehr ähnlich sind, und den von Hemingway et al. (Chemical Abstracts, Band 82, 1975, Seite 274, Zusammenfassung 135 680 k) angegebenen Alkaloiden, die in südamerikanischen Uncaria-Arten enthalten sind, bestand die Möglichkeit, dass Alkaloide die den drei ohne direkten Zusammenhang stehenden Verwendungsmöglichkeiten der Wurzelextrakte zugrundeliegenden Wirksubstanzen sein könnten.

Verschiedenen Oxindolalkaloide, die auch in Uncaria-Arten enthalten sind, werden bereits therapeutische Wirkungen zugesprochen. So wird von Chemical Abstracts, Band 92, 1980, Seite 59, Zusammenfassung 191363c über einen hypotonischen Effekt des Rohalkaloidgemisches von Uncaria rhynchophylla und Rhynchophyllin berichtet. Ebenfalls hypotonisch wirksam ist Uncarin A, wie aus Chemical Abstracts, Band 90, 1979, Seite 75, Zusammenfassung 115 313e, sowie aus Chemical Abstracts, Band 91, 1979, Seite 53, Zusammenfassung 102 109p bekannt ist. Weitere Indolalkaloide, wie Isorhynchophyllin, Hirsutin, usw. weisen Einflüsse auf neuromuskuläre und parasympathische Übertragungen auf, wie es in Chemical Abstracts, Band 84, 1976, Seite 50, Zusammenfassung 144859, und Band 91, 1979, Seite 55, Zusammenfassung 49608x erwähnt ist. Schliesslich beschreiben auch die GB-A-1056 537 und 1056 863 Alkaloide, wie Speciofolin und Rhynchocilin als hustenstillende Wirkstoffe. Aus Überlegungen heraus, dass die verschiedenen Wirkungen der oben erwähnten Wurzelextrakte auf eine unspezifische Immunsystemstimulierung zurückzuführen sein könnten, wurden aus verschiedenen Pflanzenteilen südamerikanischer Uncaria-Arten die enthaltenen Alkaloide isoliert und auf ihre immunologische Wirkung untersucht. Dabei konnten besondere immunsystemstimulierende Eigenschaften sowohl bei den Rohalkaloidfraktionen als auch bei einigen der einzeln isolierten Reinalkaloide nachgewiesen werden, sodass auch derartige Alkaloide aus anderen Uncaria-Arten sowie Alkaloide anderer Art aus verschiedenen Pflanzen hinsichtlich ihrer immunologischen Eigenschaften untersucht wurden. Die gewonnenen Ergebnisse konnten dadurch untermauert werden, wobei gleichzeitig auch die Cytotoxizität der Substanzen mit guter immunstimulierender Wirkung von Interesse war.

Zur Prüfung der verschiedenen Substanzen auf immunstimulierende und cytotoxische Eigenschaften wurden nachfolgende in-vitro- und in invivo-Testmodelle erstellt:

1. Granulozytentest modifiziert nach BRANDT

Neutrophile Granulozyten sind zusammen mit Makrophagen die wichtigsten Effektorzellen der Phagozytose und nehmen damit eine wichtige Stellung in der Infektabwehr ein. Sie stellen etwa 60-70% aller weissen Blutkörperchen im menschlichen Blut dar. Der Phagozytoseleistung dieser Granulozyten lässt sich in vitro nach einer von BRANDT (Scand. J. Haematol. Suppl 2, 1967) entwickelten und von TYMPNER (Münchn. Med. WSchr. 8, 251, 1978) modifizierten Methodik messen.

Dabei wird aus dem Blut gesunder Probanden eine Granulozytenfraktion (PMNL-Fraktion) gewonnen.

Diese PMNL-Fraktion besteht hauptsächlich aus neutrophilen Granulozyten neben wenig eosinophilen und basophilen Granulozyten sowie Lymphozyten, Monozyten und Thrombozyten. Nach Zusatz einer definierten Menge von Hefezellen (Saccharomyces cerevisiae) und der zu testenden Substanz wird zunächst 10 min bei 37° C inkubiert, die Aufschwemmung auf einem Objektträger ausgestrichen und weitere 35 min bei Raumtemperatur inkubiert. Anschliessend werden die Ausstriche angefärbt und mikroskopisch ausgewertet. Pro Objektträger werden 100 PMNL-Zellen ausgewählt und bestimmt, wieviele Hefepartikel von den einzelnen Zellen phagozytiert wurden. Der Phagozytoseindex (PJ) gibt die durchschnittliche Anzahl von phagozytierten Hefepartikeln pro Granulocyt an. Die Steigerung (S) der Phagozytoseaktivität im Blindwert wurde berechnet nach:

$$S = \frac{{}^{PJ}\text{Substanz} - {}^{PJ}\text{Kontrolle}}{{}^{PJ}\text{Kontrolle}} \times 100\ (\%)$$

Da der Test einen Teil des immunologischen Gesamtgeschehens simuliert, kann davon ausgegangen werden, dass die getesteten Substanzen auch in vivo wirksam sind, vorausgesetzt, dass sie unverändert an den Wirkort gelangen. Für Aristolochiasäure als niedermolekulare Verbindung konnte dies wie erwähnt bereits bewiesen werden. Über den dabei ablaufenden Mechanismus ist allerdings noch wenig bekannt.

2. Chemilumineszenztest (CL)

Obwohl der Granulozytentest nach BRANDT eine zuverlässige Methode zur Ermittlung phagozytosesteigernder Eigenschaften von Substanzen und Extrakten darstellt, ist er sehr zeit- und personalaufwendig. Eine Alternative bietet die neue Methode der Chemilumineszenzmessung phagozytierender Zellen.

Seitdem ALLEN et al. (Biochem. Biophys. Res. Commun, 47, 129, 1972) als erste die Lichtemission von neutrophilen Granulozyten während der Phagozytose von Latexpartikeln und verschiedener Bakterien entdeckten und NELSON et al. (Infect. Immun 14, 129, 1976) und WEIDEMANN et al. (FEBS Letters 89, 136, 1978) CL-Erscheinungen an phagozytierenden Blutmakrophagen (Monozyten) nachweisen konnten, wird diese Reaktion auch in der experimentellen und klinischen Immunologie angewendet. Der genaue Mechanismus der dabei ablaufenden Reaktion konnte allerdings bis heute noch nicht vollständig aufgeklärt werden. Zusammenfassend lässt sich die bei aktivierten Phagozyten auftretende CL folgendermassen darstellen: der Kontakt dieser Zellen mit Bakterien, Viren oder anderem körperfremden Material, z.B. auch chemische Substanzen, führt zunächst zu einer gesteigerten Sauerstoffaufnahme. Anschliessend werden in der Zelle aus dem molekularen Sauerstoff Superoxid-Anion-Radikale $O_2^-$ gebildet, die sich dann entweder spontan oder enzymatisch katalysiert in andere aktivierte Sauerstoffmoleküle, wie Wasserstoffperoxid ($H_2O_2$), Singulett-Sauerstoff ($^1O_2$), Hydroxylradikale ($\cdot OH$) und Hypochlorit-Anionen ($OCl^-$) umwandeln. Dieser Vorgang wird «respiratory burst» genannt.

Durch die Freisetzung der aktivierten Sauerstoffmoleküle in die unmittelbare Umgebung der phagozytierenden Zelle, wie auch in die phagozytische Vakuole selbst und durch die Aktivierung lysosomaler Enzyme können eingedrungene Mikroorganismen oder Tumorzellen geschädigt oder getötet werden. Auch andere Moleküle sind daran beteiligt, so z.B. sogenannte kationische Proteine und Laktoferrin. Die Zellwände der Mikroorganismen werden durch Oxidation oder Reduktion angegriffen und teilweise zerstört. Als Nebeneffekt tritt dabei CL auf, da das Zellwandmaterial als chemilumineszierendes Substrat für die aktivierten Sauerstoffmoleküle dient.

Die geringe Lichtausbeute solcher CL-Reaktionen phagozytierender Zellen wird im in-vitro-Experiment durch den Zusatz von Substanzen erhöht, die durch aktivierten Sauerstoff oxidiert werden und dabei Licht bestimmter Wellenlänge erzeugen, welches physikalisch gemessen werden kann. Als Substrate eignen sich dabei Luminol (5-Amino-2,3-dihydro-1,4-phthalazindion) und Lucigenin (10,10'-Dimethyl-9,9'-biacridiniumdinitrat), welche die CL-Antwort um das $10^3$fache steigern.

Durch den Einsatz empfindlicher Messgeräte, in denen die Photonenzählung mit Hilfe von Photomultipliern erfolgt, lässt sich die durch die oben genannten Substanzen verstärkte CL messen. Es wurde ein 6-Kanal-CL-Gerät Biolumat® LB 9505 der Fa. Berthold benutzt, das sechs Testungen zur gleichen Zeit ermöglicht. Darüberhinaus erlaubte es das verwendete Gerät auch, die CL-Antwort zeitabhängig zu registrieren. So konnten den Dosis-Wirkungs-Kurven als dritte Dimension die zeitabhängige Wirkung hinzugefügt werden.

Zur in-vitro-Untersuchung des phagozytosesteigenden Wirkung von Substanzen können entweder gereinigte Zellpopulationen von Granulozyten oder Makrophagen verwendet werden, oder die Messung erfolgt direkt in verdünntem Vollblut. Die Aktivierung der Phagozyten erfolgt dabei durch feste Partikel, z.B. opsonierte Bakterien, Zymosan® und Latexpartikel, oder durch bestimmte gelöste Substanzen. Bekannte Aktivatoren sind z.B. verschiedene Ionophoren und Fluorid-Ionen.

Durchführung des Tests

a) Vollblut: 460 µl Veronal®-gepufferte Kochsalzlösung
200 µl verdünntes Vollblut
100 µl Lucigenin-Lösung
200 µl in PBS (phosphat-gepufferte Kochsalzlösung) gelöste Substanz bzw. PBS als Kontrolle

b) PMNL: 630 µl Veronal®-gepufferte Kochsalzlösung
15 µl PMNL
100 µl Lucigenin-Lösung

200 µl in PBS gelöste Substanz bzw. PBS als Kontrolle

Nach 10minütiger Vorinkubation bei Raumtemperatur und anschliessender 10minütiger Inkubation bei 37° C im 6-Kanal-CL-Gerät wurde 40 µl opsoniertes Zymosan® unter kräftigem Umrühren zugegeben und die Messung gestartet. Messende war meistens nach 60 min. Die CL-Antowort jeder Probe wurde über 60 min beobachtet und die Messwerte in cpm graphisch dargestellt. Dabei ergibt sich ein für jede Substanz typischer Kurvenverlauf. Die integrierten Flächen sind der Intensität der CL-Antwort direkt proportional. Zur Berechnung der erfolgten Stimulation des „respiratory burst" von Granulozyten lässt sich die gleiche Formel wie beim Granulozytentest verwenden.

Für die CL-Untersuchungen an Reinsubstanzen wurde das Na-Salz der Aristiolochiasäure ausgewählt, da für diese Verbindung die phagozytosesteigernde Wirkung gesichert ist und bereits Konzentrations-Wirkungs-Daten aus dem Granulozytentest vorlagen.

Vergleich der CL-Methode mit dem Granulozytentest: wie im Granulozytentest war auch im CL-Test mit isolierten Granulozyten eine dosisabhängige Steigerung der Phagozytoseaktivität in Form der „respiratory burst" Reaktion feststellbar. Die im Granulozyten- und CL-Test nach der gleichen Formel errechneten Prozentzahlen können zwar nicht direkt miteinander verglichen werden, da die Messprinzipien beider Verfahren unterschiedlich sind, jedoch zeigt ein Vergleich der Dosis-Wirkungs-Kurven von Aristolochiasäure im Granulozyten- und CL-Test eine gute Übereinstimmung der Kurvenverläufe der beiden Testmethoden. Die CL-Methode mit isolierten Granulozyten stellt sowohl von der Genauigkeit als auch von der Empfindlichkeit her einen vollwertigen Ersatz für den arbeitsaufwendigeren Granulozytentest nach BRANDT dar, wobei durch die Erfassung der zeitabhängigen Stimulation eine noch detailliertere Beschreibung der Phagozytosevorgänge ermöglicht wird.

3. Carbon-Clearance-Test (CCT) nach BIOZZI

Mit diesem von BIOZZI et al. (C.R. Soc. Biol. 148, 431, 1954) (Progr. Liver Dis. 2, 166, 1965) entwickelten in-vivo-Test wird die Phagozytoseaktivität von Gewebsmakrophagen des Reticoloendothelialen Systems (RES) bestimmt. Dabei werden Versuchstieren gelatinestabilisierte Kohlepartikel intravenös injiziert, in bestimmten Zeitabständen Blut aus dem retroorbitalen Venenplexus entnommen und die Elimination der Kohle durch photometrische Trübungsmessung bestimmt. An der Elimination körperfremder Partikel sind die Kupffer'schen Sternzellen der Leber zu 90% und die Makrophagen der Milz zu 10% beteiligt.

Die exponentielle verlaufende Kohleelimination kann durch eine Gerade (log E gegen t) dargestellt werden, deren negative Steigung als Regressionskoeffizient K bezeichnet wird. Wird eine potentiell stimulierte Substanz eine bestimmte Zeit von Versuchsbeginn, z.B. 24 oder 48 Stunden, ein- oder mehrere Male in einer bestimmten Konzentration gegeben, so kann durch Vergleich des Regressionskoeffizienten mit dem des Blindwertes auf eine Phagozytosesteigerung geschlossen werden. Ist das Verhältnis $K_{Subst.}/K_{Kontrolle} < 1$, so besitzt die Testsubstanz eine suppressive Wirkung auf die Phagozytoseaktivität. Ist das Verhältnis $>1$, so liegt eine Stimulierung vor.

Der Regressionskoeffizient K der Kohleelimination differiert von Individuum zu Individuum in Abhängigkeit von Leber- und Milzgewicht, so dass erst nach Einbeziehung dieser Grössen die wahre Eliminationskonstante (α-Wert) ermittelt werden kann. Unsere Untersuchungen mit verschiedenen Substanzen haben dabei gezeigt, dass die Quotienten der α-Werte kaum von den Quotienten der K-Werte abweichen.

Eine Tuschesuspension mit einem 10%igen Gehalt an Gasruss (Teilchengrösse 20-25 nm) wurde mit 1%iger Gelatine in 0,9% NaCl auf 16 mg Kohle pro ml eingestellt und auf 37° C vorgewärmt. Jede Maus (-weibliche NMRI-Mäuse, 25-30 g) erhielt 0,1 ml/10 g in die Schwanzvene injiziert. Nach 3, 6, 9, 12 und 15 Minuten wurden durch Punktion des retroorbitalen Venenplexus 25 µl Blut entnommen (heparinisierte Einmal-Mikro-Pipetten). Das Blut wurde in 2 ml Aqua dest. hämolysiert und die Kohlekonzentration bei $\lambda = 650$ nm photometrisch bestimmt (Hitachi®-Spectrometer 181, Küvettendicke 1 cm).

Auswertung: Für die exponentiell verlaufende Kohleelimination wurde der Regressionskoeffizient K rechnerisch ermittelt (log E gegen t). Durch Bildung des Quotienten $D_{Subst.}/K_{Kontrolle}$ konnte auf die Stimulierung oder Suppression des RES geschlossen werden.

4. Lymphozytentransformationstest

Im Lymphozytentransformationstest wird die Induktion der Blasten-Transformation normaler Lymphozyten und deren anschliessende Mitose gemessen. Dazu wird die zu untersuchende Substanz mit isolierten Lymphozyten oder mit verdünntem Vollblut inkubiert, die Proben mit $^{14}C$- bzw. $^{3}H$-Thymidin markiert und erneut inkubiert. Das in die Zell-DNA eingebaute markierte Thymidin wird am Flüssigkeits-Scintillations-Spektrometer gemessen.

5. Prüfung auf Cytotoxizität durch den $^{3}H$-Thymidin-Einbautest an Asciteszellen

Da im Granulozytentest einige Substanzen in hoher Konzentration die Granulozyten lysierten, wurde auch die zellschädigende Wirkung am Modell des $^{3}H$-Thymidineinbautests in Asciteszellen geprüft, um eine mögliche Beziehung zwischen Phagozytosesteigerung und Cytotoxizität auffinden zu können.

In diesem in-vitro-Testsystem nach WEITZEL et al (Hoppe-Seyler's Z. Physiolog. Chem. 348, 433, 1967) kann die Cytotoxizität von Substanzen durch Hemmung der DNA-Synthese von Tumorzellen zahlenmässig durch die veränderten Einbauraten von $^{3}H$-Tymidin in Asciteszellen von

Walker-250-Carcinosarkomen von Ratten erfasst werden.

Die Ergebnisse wurden mit der Kontrolle verglichen und die Hemmeffekte in Prozent ausgedrückt.

Relativierung der Testergebnisse

Da die prozentuale Einbauhemmung bei gleichen Substanzen Schwankungen bis zu 30% zwischen einzelnen Versuchen zeigte, wurde Methotrexat®, welches als Folsäureantagonist die DNA-Synthese hemmt, als Bezugssubstanz in jeder Versuchsreihe mitgetestet. Der Mittelwert der Methotrexat®-Hemmung (43% bei 2 mg/ml) wurde zur Ermittlung der relativen Hemmung H der Substanzen herangezogen.

$$^{H}\text{Subst.} = \frac{^{H}\text{Subst. Test}}{^{H}\text{Meth. Test}} \times {^{H}\text{Meth. Mittelwert}}\ (\%)$$

Durch die Gegenüberstellung der gefundenen Werte mit den Ergebnissen des Granulozytentests konnte die auch bei anderen niedermolekularen Verbindungen gemachte Beobachtung bestätigt werden, wonach in hoher Konzentration cytotoxisch wirkende Substanzen bei weiterer Verdünnung auf $10^{-2}$ oder $10^{-3}$% stimulierend auf die Phagozytoseleistung isolierter Granulozyten wirken. Inwieweit sich allerdings diese Aussage verallgemeinen lässt, und ob sie auch auf höhermolekulare Verbindungen übertragbar ist, muss durch weitere Untersuchungen geklärt werden.

6. Akute Toxizität

Versuchstiere: weibliche oder männliche NMRI-Mäuse, 30-35 g

Haltung und Fütterung: bei 20°-22° C in Makrolonkäfigen,

SFI-Standardfutter, Wasser ad libitum

Kollektivgrösse: 5 Mäuse

Testdurchführung: Die Mäuse erhielten in $H_2O$ oder 0,9%iger NaCl-Lösung gelösten Testsubstanzen o.o. mittels einer Schlundsonde oder i.p. appliziert. Man beobachtete das Verhalten gegenüber einer Kontrollgruppe über 14 Tage.

Voraussetzung bei allen Testungen ist eine klare Löslichkeit des Testmaterials. Bei der Untersuchung von lipophilen Extrakten und Substanzen dienen die als Lösungsvermittler zugesetzten Verbindungen, wie Carboxymethylcellulose oder Dimethylsulfoxid (DMSO), in den jeweils getesteten Konzentrationen als Bezugswert, da die Lösungsmittler teilweise selbst stimulieren. Im CCT ist von dem Einsatz von DMSO wegen der hohen Toxizität abzuraten.

Ausserdem sollte die Testlösung möglichst keimarm zubereitet werden. So enthalten nämlich grampositive Bakterien in ihrer Zellwand Polysaccharide, die teilweise stimulierend auf die Phagozytoseleistung von Granulozyten wirken können. Auch die Lipopolysaccharide aus den Zellwänden gramnegativer Bakterien, sogenannte Endotoxine, beeinflussen möglicherweise das Testergebnis. Lebende Bakterien und Bakterienextrakte zeigen eine starke RES-stimulierende Wirkung. Daneben besitzen einige Endotoxine Nebenwirkungen, wie Pyrogenität, Antigenität und toxische Stoffwechselveränderungen, die sich ebenfalls störend im CCT bemerkmar machen können.

Es empfiehlt sich daher, die Testlösungen steril zu filtrieren und im Tiefkühlschrank aufzubewahren.

Zur Lokalisation des Wirkprinzipes wurden Wasser- und Ethanolextrakte von unbehandeltem und mit $NH_3$ alkalisiertem Pulver aus Uncaria tomentosa, wobei vorher eine Stimulation durch $NH_3$ allein ausgeschlossen werden konnte, weiters aus Uncaria tomentosa gewonnene Rohalkaloidgemische, daraus isolierte Reinalkaloide sowie andere Oxindolalkaloide getestet.

Zur Herstellung der Extrakte wurde die pulverisierte, getrocknete bzw. mit 10% $NH_3$ alkalisierte Droge mit dem Extraktionsmittel im Verhältnis 1:10 versetzt, 2 Tage bei Raumtemperatur am Magnetrührer extrahiert, filtriert und das Filtrat zur Trockene eingeengt.

Die Gewinnung des Rohalkaloidgemisches wurde nach folgender Methode vorgenommen. Das getrocknete Herbarmaterial wurde mit 10%igen $NH_3$ durchfeuchtet, 1 Stde. bei Zimmertemperatur stehen gelassen und unter IR-Licht schonend getrocknet. Anschliessend wurde die Droge mit Ethylacetat über vier Tage am Magnetrührer mazeriert. Der so gewonnene Rohextrakt wurde eingeengt und solange mit 2%iger $H_2SO_4$ ausgeschüttelt, bis die wässerige Phase keine positive Valser-Reaktion mehr zeigte. Die wässerige Phase wurde dann bis zur alkalischen Reaktion bei pH 8-9 mit festem $Na_2CO_3$ versetzt. Durch mehrmaliges Ausschütteln der wässerigen Phase mit Ethylacetat wurden aus 750 g Droge 6,75 g eines hellbraun gefärbten Rohalkaloidgemisches, entsprechend einer Ausbeute von 0,9% erhalten. Anschliessend erfolgte die Umwandlung in die Hydrochloridform.

Aus dem Rohalkaloidgemisch wurden die einzelnen Oxindolalkaloide isoliert und identifiziert. (Vergleichsliteratur: S.R. Johns/J.A. Lamberton, Tetrahedron Letters 1966, 931.)

1. allo-Isopteropodin, Isomer A mit der Formel $C_{21}H_{24}O_4N_2$, MG: 368

Smp: 204°-209° C

DC: $R_f$ 0,73 (LM I); 0,48 (LM II); 0,83 (LM III)

HPLC: Rt 8,8 min (TS I)

$[\alpha]_D^{20}$: −85,1° (c = 0,554/$CHCl_3$)

UV (MeOH): $\lambda_{max}$ = 208, 243, 283 (sh)

MS: m/e (rel. Int.): 368 ($M^+$, 100), 223(70), 208(35), 180(14), 146(11), 145(9), 144(5), 130(25), 69(45)

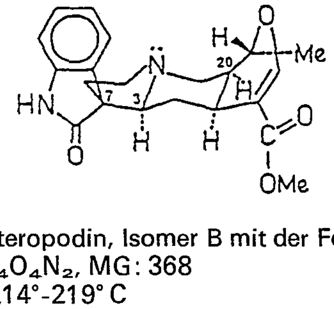

2. allo-Pteropodin, Isomer B mit der Formel $C_{21}H_{24}O_4N_2$, MG: 368

Smp: 214°-219° C

DC: $R_f$ 0,72 (LM I), 0,47 (LM II), 0,81 (LM III)
HPLC: Rt 9,8 min (TS I)
UV (MeOH): $\lambda_{max}$ = 208, 243, 283 (sh)
MS: m/e (rel. Int.); 368 ($M^+$, 100), 223(84), 208(25), 180(20), 146(8), 145(11), 144(11), 130(14), 69(35)

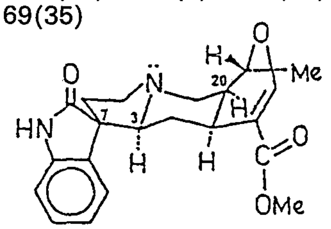

3. normal-Isomitraphyllin, Isomer A mit der Formel $C_{21}H_{24}O_4N_2$: MG 368
Smp: 96°-106° C (Z)
DC: $R_f$ 0,71 (LM I), 0,47 (LM II), 0,80 (LM III)
HPLC: Rt 9,2 min (TS I)
$[\alpha]_D^{20}$: +145° (c = 0,758/$CHCl_3$)
UV (MeOH): $\lambda_{max}$ = 208, 243, 283 (sh)
MS: m/e (rel. Int.): 368 ($M^+$, 70) 223(100), 208(10), 180(4), 146(6), 145(5), 144(7), 130(11), 69(29)

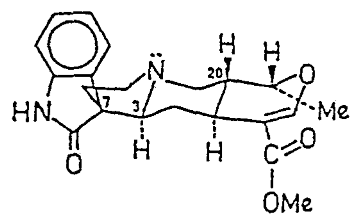

4. normal-Mitraphyllin, Isomer B mit der Formel $C_{21}H_{24}O_4N_2$, MG: 368
Smp: 264°-268° C
DC: $R_f$ 0,55 (LM I), 0,39 (LM II), 0,50 (LM III)
HPLC: Rt 10,4 min (TS I)
$[\alpha]_D^{20}$: −4,3° (c = 0,587/$CHCl_3$)
UV (MeOH): $\lambda_{max}$ = 208, 243, 283 (sh)
MS: m/e (rel. Int.): 368 ($M^+$, 60), 223(100), 208(10), 180(4), 146(6), 145(7), 144(10), 130(10), 69(27)

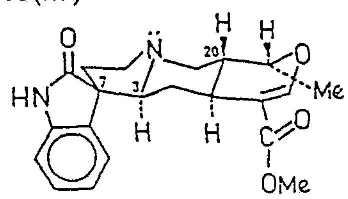

Verwendete Laufmittelsysteme (LM) für DC:
LM I Chloroform-Aceton (5:4)
LM II Chloroform-Ethanol (95:5)
LM III Ethylacetat-Isopropanol-$NH_3$ konz. (100:2:1)

Neben diesen aus Uncaria tomentosa isolierten Oxindolalkaloiden wurde das ebenfalls in Uncaria-Arten vorkommende Oxindolalkaloid Speciophyllin und das als einer der Hauptinhaltsstoffe der Wurzel von Gelsemium sempervirens (Longaniaceae) bekannte Oxindolalkaloid Gelsemin zumindest für einen Teil der immunologischen Untersuchungen herangezogen.

Zur Herstellung der wasserlöslichen Hydrochloridform wurden die freien Alkaloidbasen jeweils in Ether gelöst und HCl-Gas aus einer Gasbombe eingeleitet. Die Hydrochloride fielen weiss aus. Der Ether wurde abrotiert, die Hydrochloride nochmals mit wasserfreiem Ether gewaschen und anschliessend über Phosphorpentoxid im Vakuumexsiccator getrocknet.

Im Granulozytentest konnten nun folgende prozentuelle Phagozytosesteigerungen der verschiedenen Extrakte und Substanzen nachgewiesen werden, wobei jeweils unterschiedliche Konzentrationen verwendet wurden:

| Konzentration | $10^{-2}$ | $10^{-3}$ | $10^{-4}$ | $10^{-5}$ | $10^{-6}$ | $10^{-7}$ |
|---|---|---|---|---|---|---|
| 1. Extrakte aus Unc. tomentosa | | | | | | |
| $H_2O$-Mazerat | 7,3 | 21,8 | 24,1 | 5,7 | — | — |
| $H_2O$-Mazerat ($NH_3$) | 15,6 | 12,9 | 29,6 | 28,4 | — | — |
| EtOH-Mazerat | — | 14,4 | 12,0 | 26,3 | — | — |
| EtOH-Mazerat ($NH_3$) | 7,6 | 25,6 | 36,8 | 25,5 | — | — |
| 2. Rohalkaloidgemisch aus Unc. tom. | 20,1 | 21,0 | 15,7 | 10,3 | — | — |
| 3. Isolierte Oxindolalkaloide aus Unc. tom. | | | | | | |
| Isopteropodin | 23,4 | 48,7 | 55,0 | 38,2 | 28,2 | 13,6 |
| Pteropodin | 11,2 | 21,7 | 26,1 | 23,0 | — | — |
| Isomitraphyllin | 16,5 | 27,0 | 27,0 | 19,4 | 5,7 | — |
| Isorhynchophyllin | 10,8 | 25,2 | 27,3 | 16,1 | 8,8 | — |
| Mitraphyllin | — | — | — | — | — | — |
| Rhynchophyllin | — | — | — | — | — | — |
| 4. Andere Oxindolalkaloide | | | | | | |
| Speciophyllin | 10,5 | 18,5 | 26,0 | 35,3 | — | — |
| Gelsemin | 22,1 | 40,4 | 33,7 | 8,2 | — | — |

In der Konzentration $10^{-1}$% wurde meist Cytotoxizität festgestellt.

Zu Vergleichszwecken wurde der Granulozytentest auch mit Nicht-Oxindolalkaloiden durchgeführt.

Das Isochinolinalkaloid Berberin, das vor allem bei viralen Augenentzündungen angewendet wird, war in Konzentrationen bis zu $10^{-4}$% unwirksam. Auch beim Vincristin (Indolalkaloid vom Aspidospermintyp) und beim Camotothecin (Chinolinalkaloid) konnten bis zu Verdünnungen von $10^{-6}$% keine Wirkungen verzeichnet werden. Diese Verbindungen sind Mitosehemmer und werden als Cancerostatika eingesetzt. Für Vincristin wurde sogar eine in-vitro-Hemmung der Makrophagen-Phagozytose bei Konzentrationen von $10^{-4}$% und $10^{-5}$% festgestellt.

Die Chinolizindinalkaloide Cytisin und Spartein, Hauptalkaloide der bei septischen Erkrankungen und Geschwüren eingesetzten ganzen Pflanzen von Baptisia tinctoria (Fabaceae) zeigten ebenfalls bis zu Konzentrationen von $10^{-4}$% keine Wirkung.

Negativ verliefen auch die Teste mit den aus Thalictrum faberi (Ranunculaceae) isolierten Bisbenzylisochinolinalkaloiden Thalifaberin, Thalifabin und Huangshanin (getestet bis $10^{-4}$%), obwohl die Pflanze in der chinesischen Volksmedizin als Antiphlogistikum und zur Behandlung von Magenkrebs eingesetzt wird.

Innerhalb der aus dem Alkaloidgemisch isolierten Reinalkaloide waren deutliche Wirkunterschiede festzustellen, obwohl die Wirkmaxima übereinstimmend im Konzentrationsbereich von $10^{-2}$%-$10^{-5}$% lagen. Die im Granulozytentest nach BRANDT gefundenen Ergebnisse wurden durch den Chemilumineszenztest weitgehend bestätigt. Die Ursache für einzelne Abweichungen zwischen den beiden Testergebnissen (z.B. bei Isorhynchophyllin) konnte noch nicht geklärt werden. Nachstehende Übersicht zeigt die prozentuelle Steigerung der CL-Antwort des Rohalkaloidgemisches und der isolierten Oxindolalkaloide von Uncaria tomentosa.

Stengelextrakte der untersuchten Uncaria-Species, die alle niedrige Alkaloidgehalte besitzen, zeigten mit durchschnittlichen Steigerungsraten zwischen 10% und 20% keine starke Phagozytoseaktivierung.

Im Gegensatz dazu wiesen die isopteropodinführenden Wurzeln Werte zwischen durchschnittlich 30% und 40% Aktivitätszuwachs auf.

| Konzentration | $10^{-2}$% | $10^{-3}$% | $10^{-4}$% | $10^{-5}$% | $10^{-6}$% | $10^{-7}$% | $10^{-8}$% |
|---|---|---|---|---|---|---|---|
| 1. Alkaloidgemisch | — | 31,5 | 35,5 | 75,2 | 33,2 | — | — |
| 2. Isolierte Alkaloide: | | | | | | | |
| Isopteropodin | — | 67,2 | 66,0 | 55,7 | 68,1 | 55,3 | 31,2 |
| Pteropodin | — | 10,6 | 10,0 | 23,7 | 15,4 | — | — |
| Isomitraphyllin | — | 15,4 | 15,7 | 36,1 | 46,7 | 18,6 | — |
| Isorhynchophyllin | — | — | — | — | — | — | — |
| Mitraphyllin | — | — | — | — | — | — | — |
| Rhynchophyllin | — | — | — | — | — | — | — |

Die Blätter mit ähnlichen hohen Alkaloidkonzentrationen wie die Wurzel boten ein differenziertes Bild. Während Exemplare der Ernte von 1983, die kein Isopteropodin führten, nur etwas höhere Stimulationswerte als die Stengel erreichten, konnten bei den 1981 geernteten Blättern wiederum Steigerungsraten zwischen 30% und 40% erhalten werden. Diese Blätter enthielten Isopteropodin in etwa gleicher Konzentration wie die Wurzeln. Neben dem Alkaloidgehalt der Droge hängt daher die Wirksamkeit auch von dem Alkaloidmuster ab, wobei die unterschiedlichen Phagozytosesteigerungsraten der Einzelalkaloide im Gesamtergebnis erkennbar wurden.

Die Untersuchung des Rohalkaloidgemisches in der wasserlöslichen Hydrochloridform erbrachte eine durchschnittliche Phagozytosesteigerung von 20%. Da diese nicht ganz so hoch wie die des ethanolischen Gesamtextraktes war, scheint den im wässerigen und ethanolischen Extrakt vorhandenen Begleitstoffen eine Adjuvans-Rolle im Sinne einer Wirkungssteigerung zuzukommen. Die Bestätigung dieser Tatsache wurde erreicht, als 10 ml einer 1%igen wässerigen Lösung eines wasserlöslichen Catechinextraktes (pH 6,0) mit 10 ml einer 0,1%igen wässerigen Lösung des Gesamtalkaloidgemisches versetzt, 24 Stunden bei Raumtemperatur am Magnetrührer gerührt, gefriergetrocknet und schliesslich im Carbon-Clearance-Test untersucht wurde.

Die Resultate aller getesteten Proben im Carbon-Clearance-Test nach BIOZZI sind in nachstehender Tabelle zusammengefasst.

Wie im Granulozytentest ist auch in diesem Test die Beteiligung der Alkaloide an der Wirksamkeit erkennbar. Ein alkaloidhaltiges wässeriges Mazerat stimuliert das RES stärker als ein alkaloidarmes wässeriges Mazerat. Wurde das Alkaloidgemisch zusammen mit Catechin, das selbst nicht stimuliert, appliziert, ergab sich eine ähnliche hohe Aktivitätssteigerung wie beim wässerigen Mazerat.

*(Tabelle auf der nächsten Seite)*

Lymphozytentransformationstest

Untersucht wurde ein wässeriger Gesamtextrakt aus Uncaria tomentosa, das Rohalkaloidgemisch aus Uncaria tomentosa und zwei Mischungen P1 und P2. P1 enthält die im Granulozytentest wirksamen Alkaloide Isopteropodin, Pteropodin, Isomitraphyllin und Isorhynchophyllin, P2 die nicht wirksamen Alkaloide Mitraphyllin und Rhynchophyllin.

| Probe | Dosis | Applikation | Regressions-koeffizient K | $K_{Subst.}/K_{Kontrolle}$ |
|---|---|---|---|---|
| A | 10 mg/kg | i.p. 1×24 Std. vor Testbeginn | −0.050 | 2.3 |
| B | 10 mg/kg | i.p. 1×24 Std. vor Testbeginn | −0.046 | 1.7 |
| C | a) 0.1 mg/kg<br>b) 0.001 mg/kg | i.p., 5× letzte Dosis 24 Std. vor Testbeginn | a) −0.017<br>b) −0.037 | a) 0.3<br>b) 0.7 |
| D | 10 mg + 1 mg/kg | i.p. 1×24 Std. vor Testbeginn | −0.060 | 2.2 |
| E | 10 mg/kg | i.p. 1×24 Std. vor Testbeginn | −0.021 | 1.0 |

A = alkaloidhaltiges, wässeriges Mazerat aus Unc. tom.
B = alkaloidarmes, wässeriges Mazerat aus Unc. tom.
C = Alkaloidgemisch-HCl
D = Alkaloidgemisch zusammen mit einem wässerigen Catechinextrakt
E = wässeriger Catechinextrakt

Bei keiner der untersuchten Substanzen konnte weder bei der Vollblutmethode noch bei der Messung mit isolierten Lymphozyten eine über die Spontantransformation hinausgehende Steigerung der $^3H$-Thymidin-Einlagerung beobachtet werden.

Akute Toxizität, Cytotoxizität und Antitumorwirkung

Die Prüfung auf akute Toxizität erfolgte mit einem wässerigen Gesamtextrakt von Uncaria tomentosa und einem Rohalkaloidgemisch an Mäusen. Der Extrakt wurde mit einer Maximalkonzentration von 5 g/kg Körpergewicht peroral und mit einer Maximalkonzentration von 2 g/kg intraperitoneal appliziert. Das Alkaloidgemisch gab man oral bis zu 2 g/kg und intraperitoneal bis zu 1 g/kg. Die Mäuse wurden über einen Zeitraum von vierzehn Tagen beobachtet und dabei keinerlei Abweichung vom normalen Verhalten festgestellt.

Die Testung auf Cytotoxizität wurde mit dem $^3H$-Thymidin-Einbautest an Asciteszellen durchgeführt. Untersucht wurden verschiedene Extrakte und Alkaloidanreicherungen.

Prozentuelle Einbauhemmung von Extrakten und Alkaloidanreicherungen aus Uncaria tomentosa im $^3H$-Thymidin-Einbautest

a) Vergleich der Cytotoxizität bei einer Dosierung von 0,2%

| Probe | relative Einbau-hemmung (%) |
|---|---|
| $H_2O$-Extrakt | 42 |
| $H_2O$-Extrakt der alk. Droge | 65 |
| MeOH-Extrakt | 52 |
| Me-OH-Extrakt der alk. Droge | 64 |
| Gesamtalkaloidgemisch | 70 |
| P1-HCl | 100 |
| P2-HCl | 69 |

b) Prozentuale Einbauhemmung bei verschiedenen Konzentrationen

| Alkaloid-HCl | $10^{-1}$% | $10^{-2}$% | $10^{-3}$% | $10^{-4}$% | $10^{-5}$% |
|---|---|---|---|---|---|
| Gesamtalkaloid-gemisch | 67 | 36 | 38 | 12 | — |
| P1 | 93 | 86 | 45 | 15 | 15 |
| P2 | 64 | 58 | 20 | — | — |

Wie daraus ersichtlich, wirken die Alkaloidanreicherungen in Konzentrationen von $10^{-1}$% cytotoxisch. Die Toxizität nimmt dann aber mit steigender Verdünnung ab.

Diese Ergebnisse korellieren mit den Cytotoxizitätsuntersuchungen der Alkaloidanreicherungen P1 und P2, bei denen als Testmodelle KB- und P 388-Zellen dienten. In Konzentrationen bis zu 5 µg/ml wurde das Wachstum beider Zelltypen nicht beeinflusst. Erst bei einer Dosierung von 50 µg/ml ($5 \times 10^{-3}$%) wirkten beide Proben etwas cytotoxisch:

Cytotoxizität von P1 und P2 auf KB- und P 388-Zellen

| Alkaloidgemisch | 0,5 (µg/ml) | | 5,0 (µg/ml) | | 50 (µg/ml) | |
|---|---|---|---|---|---|---|
| | KB | P 388 | KB | P 388 | KB | P 388 |
| P1-HCl | 100 | 100 | 100 | 100 | 77 | 76 |
| P2-HCl | 100 | 100 | 100 | 100 | 77 | 64 |
| angegeben ist die prozentuale Überlebensrate der Zellen | | | | | | |

Beide Cytotoxizitätsteste bestätigen die Korrelation zwischen Cytotoxizität und Immunstimulation. So lysiert z.B. das Gesamtalkaloidgemisch in einer Konzentration von $10^{-1}$% die Granulozyten. In weiteren Verdünnungen, die nicht oder kaum mehr cytotoxisch wirken, kann man Immunstimulation beobachten. Interessanterweise ist das Gemisch P2 mit den im Granulozytentest nicht wirksamen Alkaloiden Mitraphyllin und Rhynchophyllin im $^3$H-Thymidin-Einbautest weniger toxisch als das Gemisch P1 mit den wirksamen Alkaloiden.

## Patentansprüche

1. Verwendung zumindest eines der pentacyclischen Oxindolalkaloide Isopteropodin, Pteropodin, Isomitraphyllin und Mitraphyllin zur Herstellung von Immunsystemstimulierenden Präparaten.

2. Verwendung nach Anspruch 1, dadurch gekennzeichnet, dass zumindest eines der Oxindolalkaloide ein Isomer der allo-Serie ist.

3. Präparat mit zumindest einem der pentacyclischen Oxindolalkaloide nach Anspruch 1 oder 2 zur Verwendung als Arzneimittel, wobei ein weitgehend gerbstoffreier Extrakt aus Wurzelteilen von Uncaria Tomentosa (Willd.) DC, deren frischer Bast gelbbraun oder dunkelrot gefärbt ist, ausgenommen ist.

4. Präparat nach Anspruch 3, dadurch gekennzeichnet, dass zumindest eines der nachstehenden Oxindolalkaloide enthalten ist:

allo-Isoteropodin, Isomer A mit der Formel

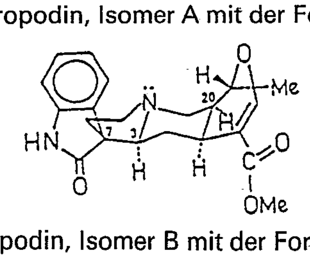

allo-Pteropodin, Isomer B mit der Formel:

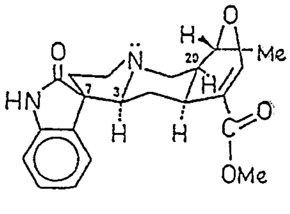

normal-Isomitraphyillin, Isomer A mit der Formel:

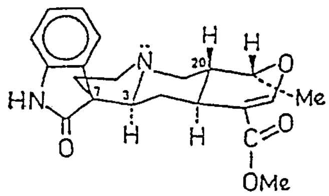

normal-Mitraphyllin, Isomer B mit der Formel:

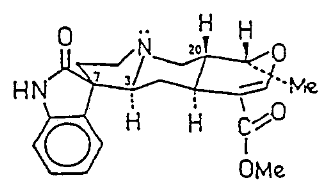

5. Präparat nach Anspruch 3, dadurch gekennzeichnet, dass jedes Oxindolalkaloid in der Hydrochloridform in wässeriger Lösung mit einer Konzentration von $10^{-3}$% bis $10^{-6}$% vorliegt.

6. Präparat nach einem der Ansprüche 3-5, dadurch gekennzeichnet, dass zumindest eines der Oxindolalkaloide in einem Alkaloid-Catechinkomplex vorliegt.

7. Präparat nach Anspruch 6, dadurch gekennzeichnet, dass einem Volumensteil der wässerigen Oxindolalkaloidlösung 10 Volumsteile eines wässerigen Catechinextraktes beigemischt ist.

## Claims

1. Use of at least one of the pentacyclic oxindole alkaloids isopteropodine, pteropodine, isomitraphylline and mitraphylline for the production of preparations stimulating the immunologic system.

2. Use according to claim 1, characterized in that at least one oxindole alkaloid is an isomer of the allo-series.

3. Preparation comprising at least one pentacyclic oxindole alkaloid according to claim 1 or 2 for use as a pharmaceutical agent, with the exclusion of an extract substantially free of tanning substances from root parts of Uncaria tomentosa (Willd.) DC having a yellow-brown or dark red fresh bast.

4. Preparation according to claim 3, characterized in that it contains at least one of the following oxindole alkaloids:
allo-isopteropodine, isomer A having the formula

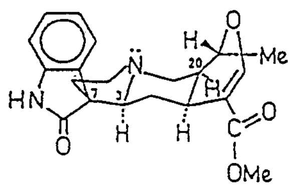

allo-pteropodine, isomer B having the formula

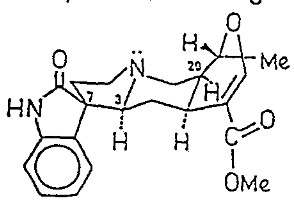

normal-isomitraphylline, isomer A having the formula

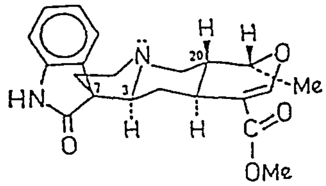

normal-mitraphylline, isomer B having the formula

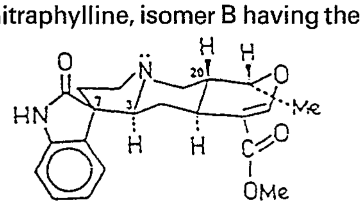

5. Preparation according to claim 3, characterized in that each oxindole alkaloid is in its hydrochloride form in aqueous solution having a concentration of $10^{-3}\%$ to $10^{-6}\%$.

6. Preparation according to one of claims 3 to 5, characterized in that at least one oxindole alkaloid is an alkaloid-catechine complex.

7. Preparation according to claim 6, characterized in that 10 parts by volume of an aqueous catechine extract are admixed to one part by volume of the aqueous oxindole alkaloid solution.

## Revendications

1. Utilisation de l'un au moins des alcaloïdes oxindoliques pentacycliques isoptéropodine, ptéropodine, isomitraphylline et mitraphylline pour la préparation de compositions stimulant le système immunitaire.

2. Utilisation selon la revendication 1, caractérisée en ce que l'un au moins des alcaloïdes oxindoliques est un isomère de la série allo.

3. Composition contenant au moins un des alcaloïdes oxindoliques pentacycliques selon la revendication 1 ou 2, pour l'utilisation en tant que médicament, à l'exclusion d'un extrait pratiquement exempt de tannin de parties de racines d'Uncaria Tomentosa (Willd.) DC dont le liber frais a une coloration brun-jaune ou rouge sombre.

4. Composition selon la revendication 3, caractérisée en ce qu'elle contient au moins un des alcaloïdes oxindoliques suivants:
l'allo-isoptéropodine, isomère A de formule:

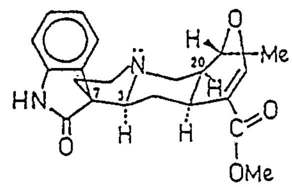

l'allo-ptéropodine, isomère B de formule:

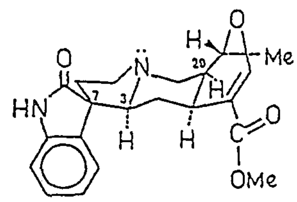

l'isomitraphylline normale, isomère A de formule:

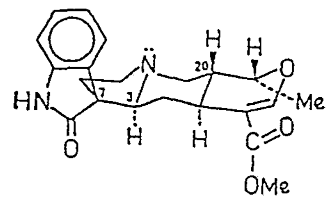

la mitraphylline normale, isomère B de formule:

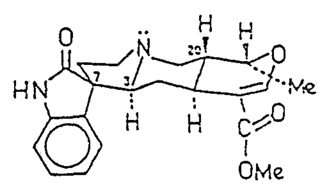

5. Composition selon la revendication 3, caractérisée en ce que chaque alcaloïde oxindolique est présent à l'état de chlorhydrate en solution aqueuse à une concentration de $10^{-3}\%$ à $10^{-6}\%$.

6. Composition selon l'une des revendications 3 à 5, caractérisée en ce que l'un au moins des alcaloïdes oxindoliques est à l'état de complexe alcaloïde-catéchine.

7. Composition selon la revendication 6, caractérisée en ce que l'on a mélangé 10 parties en volume d'un extrait aqueux de catéchine à 1 partie en volume de la solution aqueuse d'alcaloïde oxindolique.